(19) Europäisches Patentamt
European Patent Office
Office européen des brevets

(11) EP 1 147 763 A1

(12) **DEMANDE DE BREVET EUROPEEN**

(43) Date de publication:
**24.10.2001 Bulletin 2001/43**

(51) Int Cl.[7]: **A61K 7/13**

(21) Numéro de dépôt: **01400882.5**

(22) Date de dépôt: **05.04.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **18.04.2000 FR 0004994**

(71) Demandeur: **L'OREAL**
**75008 Paris (FR)**

(72) Inventeurs:
- **Kravtchenko, Sylvain**
  **92600 Asnières (FR)**
- **Plos, Grégory**
  **75011 Paris (FR)**

(74) Mandataire: **Miszputen, Laurent**
**L'OREAL-DPI**
**6 rue Bertrand Sincholle**
**92585 Clichy Cédex (FR)**

(54) **Composition de teinture d'oxydation des fibres kératiniques comprenant une 1-(4-aminophényl)-pyrrolidine et un système enzymatique**

(57) L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins une base d'oxydation choisie parmi les 1-(4-aminophényl)-pyrrolidines, et un système oxydant enzymatique comprenant au moins une enzyme de type oxydo-réductase ou peroxydase, ainsi que le procédé de teinture d'oxydation mettant en oeuvre cette composition.

EP 1 147 763 A1

## Description

L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins une base d'oxydation choisie parmi les 1-(4-aminophényl)-pyrrolidines, et un système oxydant enzymatique comprenant au moins une enzyme de type oxydo-réductase à 2 ou à 4 électrons ou peroxydase, ainsi que le procédé de teinture mettant en oeuvre cette composition.

Il est connu de teindre les fibres kératiniques et en particulier les cheveux humains avec des compositions tinctoriales contenant des colorants d'oxydation et notamment des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des colorants d'oxydation (bases d'oxydation et coupleurs), permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration importante des fibres kératiniques qui n'est pas toujours souhaitable.

Ainsi, il est connu de longue date et d'un usage très répandu, de teindre les cheveux de façon permanente avec des produits de couplage de la paraphénylènediamine (PPD) en présence de peroxyde d'hydrogène. Cependant, de nouvelles bases d'oxydation mieux tolérées ont été recherchées et proposées comme alternatives à la PPD. Parmi elles, la base tertiaire N,N-bis(β-hydroxyéthyl)-paraphénylènediamine a été largement utilisée dans les produits de teinture capillaire du commerce.

D'autre part, la coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant une base d'oxydation et éventuellement un coupleur, en association avec des enzymes du type oxydo-réductases à 2 électrons, telles que la pyranose-oxydase, la glucose-oxydase ou bien l'uricase, en présence d'un donneur pour lesdites enzymes.

Il a déjà également été proposé, notamment dans les demandes de brevets FR-A-2112549, FR-A-2694018, EP-A-504005, WO 95/07988, WO95/33836, WO95/33837, WO96/00290, WO97/19998, WO97/19999, WO97/37633, WO/98/40471 et le brevet US-3251742, de teindre les fibres kératiniques avec des compositions comprenant notamment un précurseur de colorant d'oxydation et une enzyme de type laccase (oxydo-réductase à 4 électrons).

Enfin, on a également proposé d'utiliser, pour teindre les fibres kératiniques, de la peroxydase en présence de faibles quantités de peroxyde d'hydrogène, comme on l'a décrit dans les brevets EP-548620, BE-775110 et US-3893803.

Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations ne donnant pas encore entière satisfaction, notamment du point de vue de leur intensité.

Or, la demanderesse vient maintenant de découvrir qu'il est possible d'obtenir de nouvelles teintures, capables de conduire à des colorations intenses en associant au moins une base d'oxydation choisie parmi les 1-(4-aminophényl)-pyrrolidines à un système oxydant enzymatique comprenant au moins une enzyme de type oxydo-réductase à 2 électrons ou à 4 électrons ou peroxydase.

Il devient également possible de diminuer la sélectivité de la teinture par rapport à la teinture obtenue notamment avec des compositions associant une base d'oxydation tertiaire des plus connues de l'art antérieur tels que la N,N-bis(β-hydroxyéthyl)-paraphénylènediamine (largement utilisé dans les produits commerciaux de la coloration capillaire), à au moins une enzyme du type décrit ci-dessus.

Ces découvertes sont à la base de la présente invention.

L'invention a donc pour premier objet une composition prête à l'emploi, pour la teinture d'oxydation des fibres kératiniques et en particulier des fibres kératiniques humaines telles que les cheveux, comprenant dans un milieu approprié pour la teinture, un système oxydant enzymatique comprenant au moins une enzyme de type oxydo-réductase à 2 électrons ou à 4 électrons ou peroxydase, au moins un précurseur de colorant d'oxydation, caractérisée par le fait que le précurseur de colorant d'oxydation est choisi parmi les 1-(4-aminophényl)-pyrrolidines de formule (I) suivante :

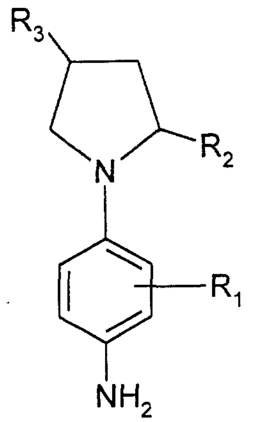

(I)

dans laquelle,

$R_1$ désigne un atome d'hydrogène, un radical alkyle en $C_1$-$C_6$, ou monohydroxyalkyle en $C_1$-$C_5$, ou polyhydroxyalkyle en $C_2$-$C_5$ ;
$R_2$ désigne un atome d'hydrogène, un radical $-CONH_2$, ou monohydroxyalkyle en $C_1$-$C_5$, ou polyhydroxyalkyle en $C_2$-$C_5$ ;
$R_3$ désigne un atome d'hydrogène, ou un radical OH ;

et leurs sels d'addition avec un acide.

Les sels d'addition avec un acide des 1-(4-aminophényl)-pyrrolidines de formule (I) sont notamment choisis parmi les chlorhydrates, les phosphates, les bromhydrates, les sulfates et les tartrates, les lactates et les acétates.

L'invention a également pour objet un procédé de teinture d'oxydation des fibres kératiniques mettant en oeuvre cette composition tinctoriale prête à l'emploi.

Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

Le ou les 1-(4-aminophényl)-pyrrolidines de formule (I) selon l'invention sont des composés bien connus de l'homme de l'art et notamment décrits et préparés dans les brevets américains N°- 5851237, 5876464, et 5993491, dans lesquels ils sont également proposés en teinture capillaire d'oxydation classique avec le peroxyde d'hydrogène comme oxydant.

Selon la présente invention, on préfère tout particulièrement utiliser des 1-(4-aminophényl)-pyrrolidines de formule (I) pour laquelle :

- $R_1$, $R_2$ et $R_3$ désignent un atome d'hydrogène; le composé de formule (I) est alors la 1-(4-aminophényl)-pyrrolidine, ou,
- R1 et $R_3$ désignent un atome d'hydrogène et $R_2$ désigne le radical $-CH_2OH$; le composé de formule (I) est alors le 1-(4-aminophényl)-2-pyrrolidineméthanol,
  ou,
- $R_1$ désigne un atome d'hydrogène, $R_2$ désigne le radical $-CH_2OH$ et $R_3$ désigne le radical OH; le composé de formule (I) est alors le 1-(4-aminophényl)-4-hydroxy-2-pyrrolidineméthanol,
  ou,
- $R_1$ et $R_3$ désignent un atome d'hydrogène et $R_2$ désigne le radical $-CONH_2$; le composé de formule (I) est alors la N-(4-aminophényl)-prolineamide.

Le ou les 1-(4-aminophényl)-pyrrolidines de formule (I) utilisés conformément à l'invention représentent de

préférence de 0,001 à 10 % en poids environ du poids total de la composition conforme à l'invention et encore plus préférentiellement de 0,01 à 8% environ de ce poids.

Système oxydant enzymatique

La ou les oxydo-réductases à 2 électrons utilisées dans la composition tinctoriale prête à l'emploi conforme à l'invention, sont utilisées en présence d'un donneur pour la ou lesdites enzymes, et peuvent notamment être choisies parmi les pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition, et encore plus préférentiellement de 0,1 à 5 % en poids environ de ce poids.

On peut aussi définir la quantité d'enzyme en fonction de son activité.

L'activité enzymatique des oxydoréductases à 2 électrons conformes à l'invention peut être définie à partir de l'oxydation du donneur en condition aérobie.

Une unité U correspond à la quantité d'enzyme conduisant à la génération d'une µmole de $H_2O_2$ par minute à un pH de 8,5 et à une température de 25°C. De façon préférentielle, la quantité d'oxydoréductase à 2 électrons conforme à l'invention est comprise entre 10 et $10^8$ unités U environ pour 100g de composition tinctoriale.

Selon l'invention, on entend par donneur, les différents substrats également nécessaires au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose; à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; à titre de donneur pour les uricases, on peut citer l'acide urique et ses sels ; à titre de donneur pour les choline oxydases, on peut citer la choline et ses sels d'addition avec un acide comme le chlorhydrate de choline, et la bétaïne aldéhyde ; à titre de donneur pour les sarcosine oxydases, on peut citer la sarcosine, la N-méthyl-L-leucine, la N-méthyl-DL-alanine, et la N-méthyl-DL-valine ; et enfin, à titre de donneur pour les bilirubine oxydases, on peut citer la bilirubine.

Le ou les donneurs (ou substrats) utilisés conformément à l'invention représentent de préférence de 0,01 à 20 % en poids environ du poids total de la composition conforme à l'invention et encore plus préférentiellement de 0,1 à 5 % en environ de ce poids.

La ou les oxydo-réductases à 4 électrons utilisées dans la composition tinctoriale prête à l'emploi conforme à l'invention peuvent notamment être choisies parmi les laccases, les tyrosinases, les catéchol oxydases et les polyphénols oxydases.

Selon une forme de réalisation particulière et préférée de l'invention la ou les oxydo-réductases à 4 électrons sont choisies parmi les laccases.

Ces laccases peuvent notamment être choisies parmi les laccases d'origine végétale, d'origine animale, d'origine fongique (levures, moisissures, champignons) ou d'origine bactérienne, les organismes d'origine pouvant être mono- ou pluri- cellulaires. Les laccases peuvent également être obtenues par biotechnologie.

Parmi les laccases d'origine végétale utilisables selon l'invention, on peut citer les laccases produites par des végétaux effectuant la synthèse chlorophyllienne telles que celles indiquées dans la demande de brevet FR-A-2 694 018.

On peut notamment citer les laccases présentes dans les extraits d'Anacardiacées tels que par exemple les extraits de Magnifera indica, de Schinus molle ou de Pleiogynium timoriense ; dans les extraits de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites

dans les demandes de brevet WO95/07988, WO95/33836, WO95/33837, WO96/00290, WO97/19998 et WO97/19999, dont le contenu fait partie intégrante de la présente description comme par exemple la ou les laccases issues de Scytalidium, de Polyporus pinsitus, de Myceliophtora thermophila, de Rhizoctonia solani, de Pyricularia orizae, et leurs variantes. On peut également citer la ou les laccases issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

On choisira plus préférentiellement les laccases d'origine fongiques, éventuellement obtenues par biotechnologie.

L'activité enzymatique des laccases utilisées conformément à l'invention et ayant la syringaldazine parmi leurs substrats peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5. L'activité enzymatique des laccases utilisées selon l'invention peut aussi être définie à partir de l'oxydation de la paraphénylènediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

De manière générale, la ou les oxydo-réductases à 4 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

De manière particulière, et lorsqu'une ou plusieurs laccases sont utilisées, la quantité de laccase(s) présente dans la composition tinctoriale prête à l'emploi conforme à l'invention variera en fonction de la nature de la ou des laccases utilisées. De façon préférentielle, la quantité de laccase(s) est comprise entre 0,5 et 2000 Lacu environ (soit entre 10000 et $40.10^6$ unités U environ ou soit entre 20 et $20.10^6$ unités ulac) pour 100 g de composition tinctoriale prête à l'emploi.

La ou les peroxydases utilisées dans la composition tinctoriale prête à l'emploi conforme à l'invention, peuvent notamment être choisies parmi les enzymes appartenant à la sous-classe 1.11.1 décrite dans l'ouvrage Enzyme Nomenclature, Academic Press Inc., 1984. Certaines d'entre elles nécessitent la présence d'un donneur pour fonctionner. C'est le cas, en particulier des NADH peroxydases (1.11.1) [donneur = NADH], des acides gras peroxydases (1.11.1.3) [donneur = acide gras, par exemple palmitate] , des NADPH peroxydases (1.11.1.2), [donneur = NADPH], des cytochrome-c peroxydases (1.11.1.5) [donneur = ferrocytochrome c], des iodures peroxydases (1.11.1.8) [donneur = iodure], des chlorures peroxydases (1.11.10) [donneur = chlorure], des L. ascorbates peroxydases (1.11.1.11) [donneur = L.ascorbate], des glutathion peroxydases (1.11.1.9) [donneur = glutathion].

D'autres peroxydases fonctionnent sans donneur autre que le colorant d'oxydation ; il en est ainsi des catalases (1.11.1.6) et des peroxydases simplex (1.11.1.7).

Selon l'invention, on utilise de préférence les peroxydases simplex (1.11.1.7).

Toutes les peroxydases fonctionnent en présence de peroxyde d'hydrogène qui est apporté tel quel ou généré in situ par voie enzymatique [oxydase(s) à 2 électrons et donneur(s) en présence d'air].

Les peroxydases utilisées peuvent être d'origine végétale, animale, fongique, bactérienne. Elles peuvent être également obtenues par biotechnologie.

Ainsi, les peroxydases peuvent par exemple être issues de la pomme, de l'abricot, de l'orge, du radis noir, de la betterave, du chou, de la carotte, du maïs, du coton, de l'ail, du raisin, de la menthe, de la rhubarbe, du soja, de l'épinard, du coprin, du lait de bovin, des microorganismes du type Acetobacter peroxidans, Staphylococcus faecalis, Arthromyces ramosus.

L'unité d'activité de la peroxydase simplex (1.11.1.7) peut se définir comme étant la quantité d'enzyme simplex formant 1mg de purpurogalline à partir du pyrogallol en 20s à pH6 et à 20°C. A titre d'exemple, la peroxydase de radis noir P6782 de SIGMA présente une activité d'environ 250 unités par mg.

La concentration d'utilisation de ce type d'enzyme varie donc entre 25 et $5.10^6$ unités pour 100g de composition.

La ou les peroxydases conformes à l'invention représentent de préférence de 0,0001 à 20% en poids environ du poids total de la composition, et encore plus préférentiellement de 0,001 à 10% en poids environ de ce poids.

Coupleurs

La composition tinctoriale prête à l'emploi conforme à l'invention peut renfermer un ou plusieurs coupleurs

choisis parmi ceux classiquement utilisés en teinture d'oxydation et notamment parmi les méta-aminophénols, les métaphénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale prête à l'emploi et encore plus préférentiellement de 0,001 à 10% environ.

Précurseurs de colorant d'oxydation additionnels

La composition tinctoriale prête à l'emploi conforme à l'invention peut également contenir des précurseurs de colorants d'oxydation autres que ceux de formule (I) selon l'invention et habituellement utilisés en teinture d'oxydation choisis parmi les paraphénylènediamines dont on peut citer notamment parmi elles, la paraphénylènediamine, la paratoluylènediamine, la 2-hydroxyéthylparaphénylènediamine, la 1-N,N-bis(2-hydroxyéthyl)-paraphénylènediamine, les para-aminophénols tels que le 3-méthyl-4-aminophénol et le 4-aminophénol, les orthophénylènes diamines, les orthoaminophénols, les bases doubles, les bases hétérocycliques comme les pyrimidines telles que la 2,4,5,6-tétraaminopyrimidine ou comme les pyrazoles tel que le 1-(2-hydroxyéthyl)-4,5-diamino-pyrazole.

Le ou les précurseurs de colorants d'oxydation additionnels peuvent être présents à une concentration comprise entre 0,0001 et 15% en poids par rapport au poids total de ladite composition.

Les sels d'addition avec un acide de ces colorants d'oxydation éventuellement présents dans les compositions tinctoriales de l'invention (bases et/ou coupleurs) sont notamment choisis parmi les chlorhydrates, les bromhydrates, les sulfates et les tartrates, les lactates et les acétates.

Colorants directs

Selon une forme de réalisation préférée, la composition tinctoriale prête à l'emploi conforme à l'invention peut en outre renfermer un ou plusieurs colorants directs notamment pour modifier les nuances en les enrichissant de reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Milieu de teinture

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ceux décrits dans la demande de brevet français 2 773 475 dont le contenu fait partie intégrante de l'invention, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale prête à l'emploi, et encore plus préférentiellement entre 5 et 30 % en poids environ.

Le pH de la composition prête à l'emploi conforme à l'invention est choisi de telle manière que l'activité enzymatique de l'oxydo-réductase à 2 électrons ou à 4 électrons ou de la peroxydase soit suffisante. Il est généralement compris entre 3 et 11 environ, et de préférence entre 4 et 9 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide

tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino 1-propanol ainsi que leurs dérivés, les hydroxydes de sodium ou de potassium et les composés de formule (II) suivante :

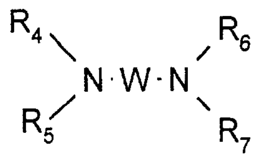

(II)

dans laquelle W est un reste propylène éventuellement substitué par un groupement hydroxyle ou un radical alkyle en $C_1$-$C_4$ ; $R_4$ $R_5$, $R_6$ et $R_7$, identiques ou différents, représentent un atome d'hydrogène, un radical alkyle en $C_1$-$C_4$ ou hydroxyalkyle en $C_1$-$C_4$.

La composition tinctoriale prête à l'emploi conforme à l'invention peut également renfermer divers adjuvants utilisés classiquement dans les compositions pour la teinture des cheveux, tels que des tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, ou ceux décrits dans la demande de brevet français 2 773 474 dont le contenu fait partie intégrante de l'invention, des agents filmogènes, des céramides, des agents conservateurs et des réducteurs, des agents opacifiants.

Parmi les agents épaississants organiques, on utilise plus particulièrement les chitosanes décrits dans la demande de brevet de la demanderesse FR-9907828 c'est à dire :

- les chitosanes salifiés par un acide organique tels que par exemple l'acide lactique, l'acide glutamique et de préférence l'acide pyrrolidone carboxylique ou parmi ceux salifiés par un acide minéral tel que par exemple l'acide chlorhydrique et l'acide sulfurique.
- les chitosanes chimiquement modifiés dont on peut notamment citer le produit vendu sous la dénomination N,O-carboxymethylchitosan par la société CHITOGENICS LTD, le carboxyméthylchitosan vendu sous la dénomination OLEVASAN par la société SINOLION, le N-carboxybutylchitosane vendu sous les dénominations commerciales CHITOLAM NB 101 ou EVALSAN par la société CHITO BIOS , le N-succinylchitosane vendu par la société CHIMEX sous la dénomination MEXOMERE PAD, ou vendu par la société FRANCECHITINE sous la dénomination KITINAMI, ou vendu par la société KATAKURA CHIKKARIN sous la dénomination SUCCINYL CHITOSAN, le N-succinylcarboxyméthylchitosane vendu sous la dénomination CHITOSOLLEN par la société IKEDA.

Lesdits chitosanes salifiés ou modifiés représentent alors de préférence de 0,01 à 20 % en poids environ par rapport au poids total de la composition tinctoriale prête à l'emploi et encore plus préférentiellement de 0,1 à 5 % en poids environ de ce poids.

La composition prête à l'emploi selon l'invention comprend en outre de préférence dans la composition contenant la ou les 1-(4-aminophényl)-pyrrolidines de formule (I), au moins un polymère épaississant de type non ionique, anionique, ou cationique comportant au moins une chaîne grasse.

Parmi les polymères épaississants comportant au moins une chaîne grasse et de type anionique, on peut citer :

- **(I)** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

$$CH_2 = C\ R'\ CH_2\ O\ B_n\ R \qquad (1)$$

dans laquelle R' désigne H ou $CH_3$, B désigne le radical éthylèneoxy, n est nul ou désigne un entier allant de 1 à

100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl ($C_{18}$).
Des polymères amphiphiles anioniques de ce type sont décrits et préparés, selon un procédé de polymérisation en émulsion, dans le brevet EP-0 216 479.
Parmi ces polymères épaississants anioniques à chaîne grasse, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

- **(II)** ceux comportant au moins un motif hydrophile de type acide carboxylique insaturé oléfinique, et au moins un motif hydrophobe de type ester d'alkyl ($C_{10}$-$C_{30}$) d'acide carboxylique insaturé.
De préférence, ces polymères sont choisis parmi ceux dont le motif hydrophile de type acide carboxylique insaturé oléfinique correspond au monomère de formule (II) suivante :

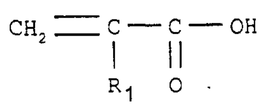

**(II)**

dans laquelle, $R_1$ désigne H ou $CH_3$ ou $C_2H_5$, c'est-à-dire des motifs acide acrylique, acide méthacrylique ou acide éthacrylique, et dont le motif hydrophobe de type ester d'alkyl ($C_{10}$-$C_{30}$) d'acide carboxylique insaturé correspond au monomère de formule (III) suivante :

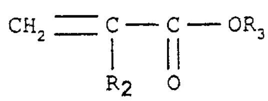

(III)

dans laquelle, $R_2$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R_3$ désignant un radical alkyle en $C_{10}$-$C_{30}$, et de préférence en $C_{12}$-$C_{22}$.
Des esters d'alkyles ($C_{10}$-$C_{30}$) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
Des polymères anioniques de ce type sont par exemple décrits et préparés, selon les brevets US-3 915 921 et 4 509 949.
Parmi ce type de polymères épaississants anioniques à chaîne grasse, on utilisera plus particulièrement des polymères formés à partir d'un mélange de monomères comprenant :

(i) essentiellement de l'acide acrylique,
(ii) un ester de formule (III) décrite ci-dessus et dans laquelle $R_2$ désigne H ou $CH_3$, $R_3$ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères épaississants anioniques à chaîne grasse, on utilisera plus particulièrement ceux

constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en $C_{10}$-$C_{30}$ (motif hydrophobe), et 0,1 à 0,6% en poids de monomère polymérisable réticulant tel que ceux décrits précedemment. Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX .

- **(III)** les terpolymères d'anhydride maléique/α-oléfine en $C_{30}$-$C_{38}$/ maléate d'alkyle tel que le produit (copolymère anhydride maléique/α-oléfine en $C_{30}$-$C_{38}$/maléate d'isopropyle) vendu sous le nom PERFORMA V 1608 par la société NEWPHASE TECHNOLOGIES.

- **(IV)** les terpolymères acryliques comprenant :

  (a) environ 20% à 70% en poids d'un acide carboxylique à insaturation α,β-monoéthylénique,
  (b) environ 20 à 80% en poids d'un monomère à insaturation α,β-monoéthylénique non-tensio-actif différent de (a),
  (c) environ 0,5 à 60% en poids d'un mono-uréthane non-ionique qui est le produit de réaction d'un tensio-actif monohydrique avec un monoisocyanate à insaturation monoéthylénique, tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.

- **(V)** les copolymères comportant parmi leurs monomères un acide carboxylique à insaturation α,β-monoéthylénique et un ester d'acide carboxylique à insaturation α,β-monoéthylénique et d'un alcool gras oxyalkyléné. Préférentiellement ces composés comprennent également comme monomère un ester d'acide carboxylique à insaturation α,β-monoéthylénique et d'alcool en C1C4.
  A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Les polymères épaississants à chaîne grasse de type non ionique, utilisés selon l'invention, sont choisis de préférence parmi :

- **(1)** les celluloses modifiées par des groupements comportant au moins une chaîne grasse ;
  on peut citer à titre d'exemple :

  - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS (alkyles en $C_{16}$) vendu par la société AQUALON, ou le produit BERMOCOLL EHM 100 vendu par la société BEROL NOBEL,
  - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.

- **(2)** les hydroxypropylguars modifiés par des groupements comportant au moins une chaîne grasse tel que le produit ESAFLOR HM 22 (chaîne alkyle en $C_{22}$) vendu par la société LAMBERTI, les produits RE210-18 (chaîne alkyle en $C_{14}$) et RE205-1 (chaîne alkyle en $C_{20}$) vendus par la société RHONE POULENC.

- **(3)** les copolymères de vinyl pyrrolidone et de monomères hydrophobes à chaîne grasse ;
  on peut citer à titre d'exemple :

  - les produits ANTARON V216 ou GANEX V216 (copolymère vinylpyrrolidone / hexadécène) vendu par la société I.S.P.
  - les produits ANTARON V220 ou GANEX V220 (copolymère vinylpyrrolidone / eicosène) vendu par la société I.S.P.

- **(4)** les copolymères de méthacrylates ou d'acrylates d'alkyles en $C_1$-$C_6$ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.

- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

- **(6)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

- **(7)** les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom. Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184, l'Aculyn 44 et l'Aculyn 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexylisocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis (4-cyclohexylisocyanate) (SMDI), à 35% en poids dans un mélange de propylèneglycol (39%) et d'eau (26%)].

On peut également citer le produit ELFACOS T210 à chaîne alkyle en $C_{12-14}$ et le produit ELFACOS T212 à chaîne alkyle en $C_{18}$ de chez AKZO.

Le produit DW 1206B de chez ROHM & HAAS à chaîne alkyle en $C_{20}$ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

<u>Les polymères épaississants à chaîne grasse de type cationique</u> utilisés selon la présente invention sont choisis de préférence parmi les dérivés de cellulose quaternisée et les polyacrylates à groupements latéraux aminés non cycliques.

Les dérivés de cellulose quaternisée sont, en particulier,

- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en $C_8$-$C_{30}$, les produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en $C_{18}$) commercialisés par la société CRODA.

Les polyacrylates à groupements latéraux aminés, quaternisés ou non, possèdent par exemple des groupements hydrophobes du type stéareth 20 (alcool stéarylique polyoxyéthyléné(20)).

Comme exemples de polyacrylates à chaînes latérales aminées, on peut citer les polymères 8781- 121B ou 9492-103 proposés par la société NATIONAL STARCH.

Les polymères épaississants à chaîne grasse de type anionique, non ionique ou cationique sont utilisés de préférence en une quantité pouvant varier d'environ 0,01 à 10% en poids du poids total de la composition de teinture. Plus préférentiellement, cette quantité varie d'environ 0,1 à 5% en poids.

La composition prête à l'emploi selon l'invention peut également comprendre en outre de préférence dans la composition contenant la ou les 1-(4-aminophényl)-pyrrolidines de formule (I), au moins un polymère cationique ou amphotère .

Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :

**(1)** Les homopolymères ou copolymères dérivés d'esters ou d'amides acryliques ou méthacryliques et comportant au moins un des motifs de formules (I), (II), (III) ou (IV) suivantes:

(I)

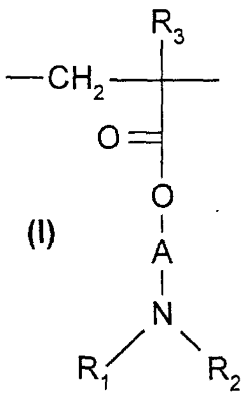

(II)

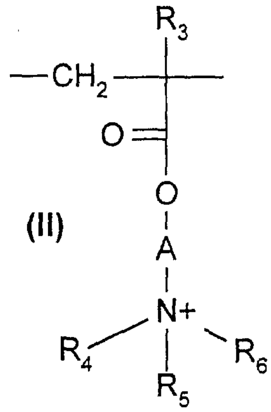

(III)

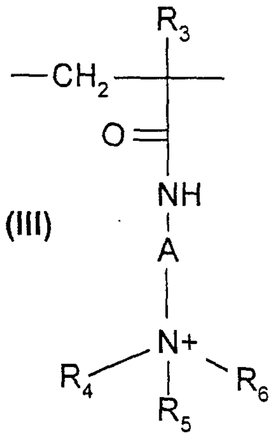

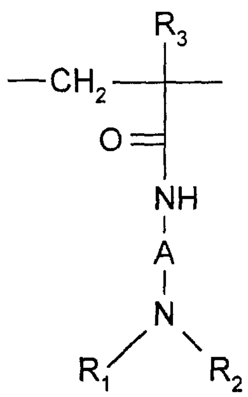

(IV)

dans lesquelles:

$R_3$, identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.
Ainsi, parmi ces polymères de la famille (1), on peut citer :

- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,

- les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,

- le copolymère d'acrylamide et de méthosulfate de méthacryloyloxyéthyltriméthylammonium vendu sous la dénomination RETEN par la société HERCULES,

- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,

- les terpolymères méthacrylate de diméthyl amino éthyle/ vinylcaprolactame/ vinylpyrrolidone tel que le produit vendu sous la dénomination GAFFIX VC 713 par la société ISP,

- les copolymère vinylpyrrolidone / méthacrylamidopropyl dimethylamine commercialisés notamment sous la dénomination STYLEZE CC 10 par ISP,

- et les copolymères vinylpyrrolidone / méthacrylamide de diméthylaminopropyle quaternisés tel que le produit vendu sous la dénomination "GAFQUAT HS 100" par la société ISP.

**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.

Les produits commercialisés répondant à cette définition sont plus particulièrement les produits vendus sous la dénomination "Celquat L 200" et "Celquat H 100" par la Société National Starch.

**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.

De tels produits sont commercialisés notamment sous les dénominations commerciales de JAGUAR C13 S, JAGUAR C 15, JAGUAR C 17 ou JAGUAR C162 par la société MEYHALL.

**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bishalohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.

**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

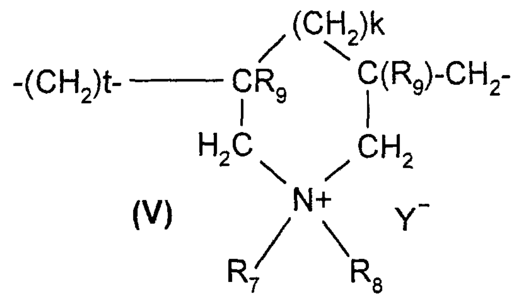

(V)

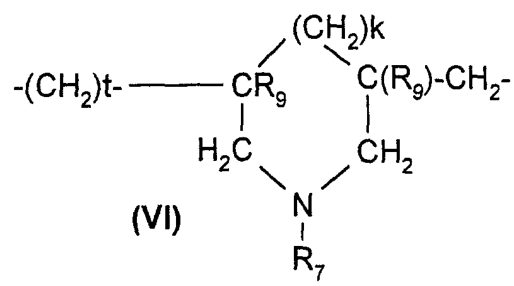

(VI)

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

**(10)** Le polymère de diammonium quaternaire contenant des motifs récurrents répondant à la formule :

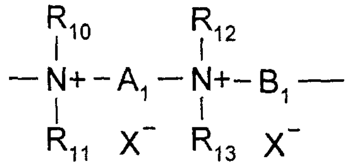

**(VII)**

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$,

$R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH- $R_{14}$-D où $R_{14}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
$X^-$ désigne un anion dérivé d'un acide minéral ou organique;
A1, $R_{10}$ et $R_{12}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement $-(CH_2)n-CO-D-OC-(CH_2)n-$ - dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH-.

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante:

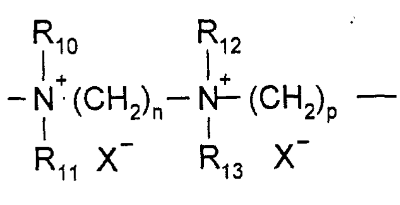

**(VIII)**

dans laquelle $R_{10}$, $R_{11}$, $R_{12}$ et $R_{13}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.
**(11)** Les polymères de polyammonium quaternaire constitués de motifs récurrents de formule (IX) :

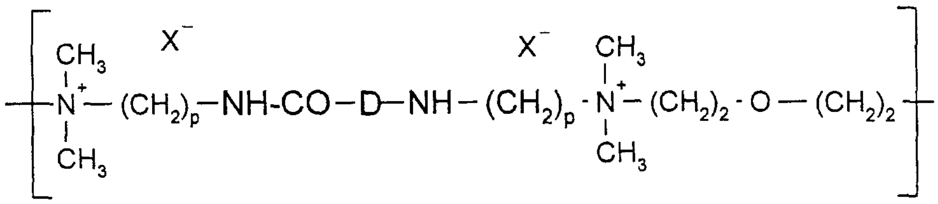 **(IX)**

dans laquelle p désigne un nombre entier variant de 1 à 6 environ, D peut être nul ou peut représenter un groupement $-(CH_2)_r$ -CO- dans lequel r désigne un nombre égal à 4 ou à 7, $X^-$ est un anion ;

De tels polymères peuvent être préparés selon les procédés décrits dans les brevets U.S.A. n° 4 157 388, 4 390 689, 4 702 906, 4 719 282. Ils sont notamment décrits dans la demande de brevet EP-A-122 324.

Parmi eux, on peut par exemple citer, les produits "Mirapol A 15", "Mirapol AD1", "Mirapol AZ1" et "Mirapol 175" vendus par la société Miranol.

**(12)** Les polymères quaternaires de vinylpyrrolidone et de vinylimidazole tels que par exemple les produits commercialisés sous les dénominations Luviquat FC 905, FC 550 et FC 370 par la société B.A.S.F.

**(13)** Les polyamines comme le Polyquart H vendu par HENKEL, référencé sous le nom de " POLYETHYLENEGLYCOL (15) TALLOW POLYAMINE " dans le dictionnaire CTFA.

**(14)** Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères de formules (W) et (U) suivantes :

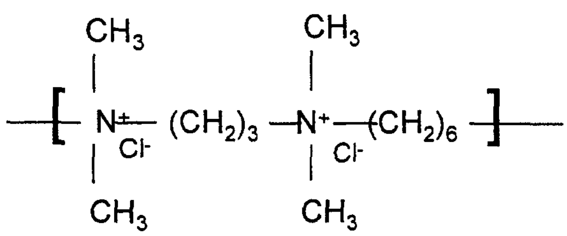 **(W)**

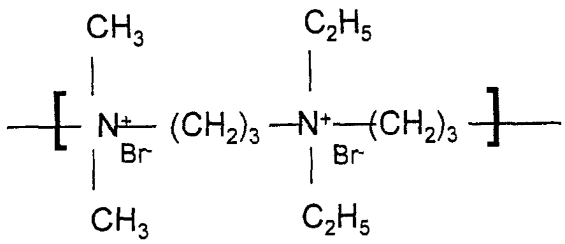 **(U)**

La concentration en polymère cationique dans les compositions selon la présente invention peut varier de 0,01 à 10% en poids par rapport au poids total de la composition, de préférence de 0,05 à 5% et plus préférentiellement encore de 0,1 à 3%.

Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes; K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.

Le composé vinylique peut être également un sel de dialkyldiallylammonium tel que le chlorure de diéthyldiallylammonium. Les copolymères d'acide acrylique et de ce dernier monomère sont proposés sous les appelations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butyl aminoéthyle, de N,N'-diméthylaminoéthyle, de N-tertio-butylaminoéthyle. On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale :

$$-\!\!\left[ CO - R_{19} - CO - Z \right]\!\!- \qquad (X)$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

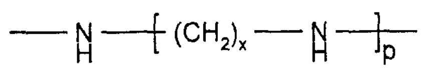

**(XI)**

où x=2 et p=2 ou 3, ou bien x=3 et p=2 ce radical dérivant de la diéthylène triamine, de la triéthylène tétraamine ou de la dipropylène triamine;
b) dans les proportions de 0 à 40 moles % le radical (XI) ci-dessus, dans lequel x=2 et p=1 et qui dérive de l'éthylènediamine, ou le radical dérivant de la pipérazine :

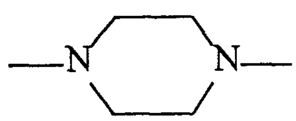

c) dans les proportions de 0 à 20 moles % le radical $-NH-(CH_2)_6-NH-$dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule

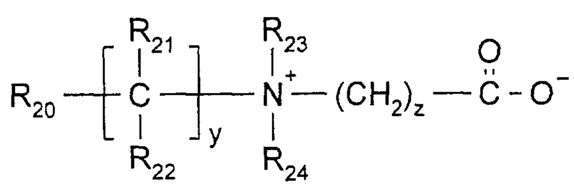

**(XII)**

dans laquelle $R_{20}$ désigne un groupement insaturé polymérisable tel qu'un groupement acrylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.
Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane comportant des motifs monomères répondant aux formules (XIII), (XIV), (XV) suivantes :

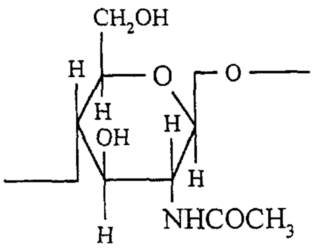

**(XIII)**

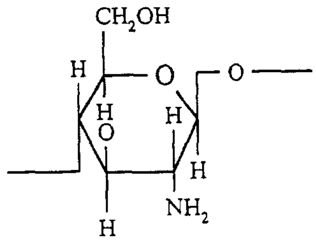

**(XIV)**

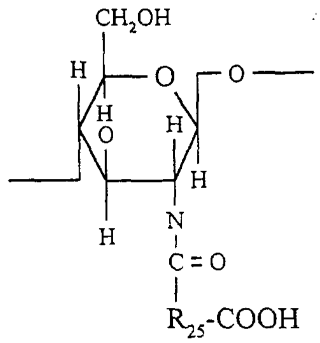

**(XV)**

le motif (XIII) étant présent dans des proportions comprises entre 0 et 30%, le motif (XIV) dans des proportions comprises entre 5 et 50% et le motif F dans des proportions comprises entre 30 et 90%, étant entendu que dans ce motif (XV), $R_{25}$ représente un radical de formule :

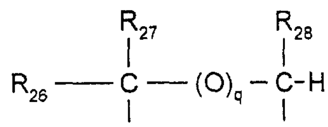

dans laquelle
si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;
ou si q=1, $R_{26}$, $R_{27}$ et $R_{28}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (XI) tels que ceux décrits par exemple dans le brevet français 1 400 366 :

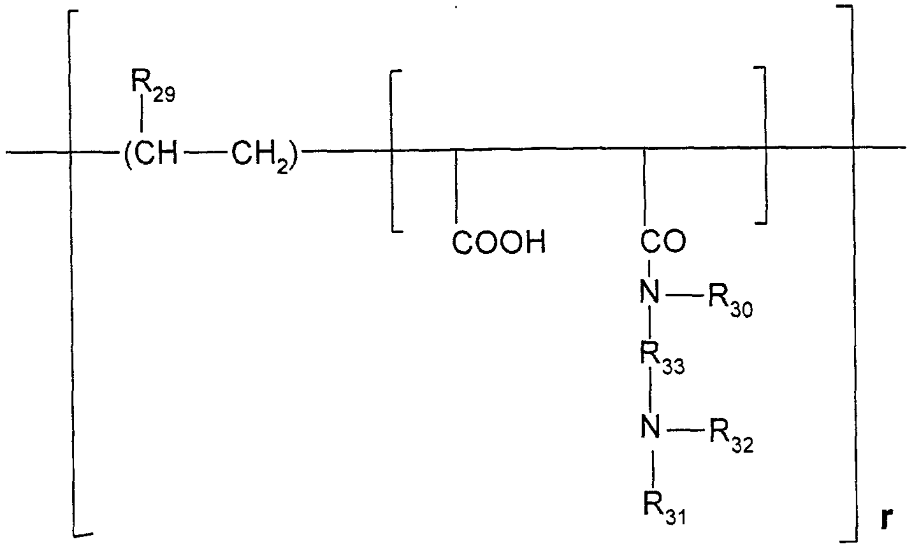

(XVI)

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$, $-CH_2-CH_2-CH_2-$, $-CH_2-CH(CH_3)-$, $R_{31}$ ayant les significations mentionnées ci-dessus,
ainsi que les homologues supérieurs de ces radicaux et contenant jusqu'à 6 atomes de carbone.

(8) Des polymères amphotères du type -D-X-D-X- choisis parmi:

a) les polymères obtenus par action de l'acide chloracétique ou le chloracétate de sodium sur les composés comportant au moins un motif de formule :

$$-D-X-D-X-D- \qquad (XVII)$$

où D désigne un radical

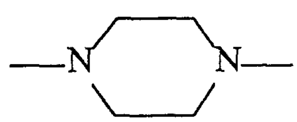

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

$$- D-X-D-X- \qquad (XVIII)$$

où D désigne un radical

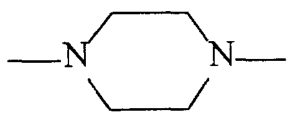

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

Les polymères amphotères particulièrement préférés selon l'invention sont ceux de la famille (1).
Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.
La composition prête à l'emploi selon l'invention comprend également de préférence dans la composition contenant la ou les 1-(4-aminophényl)-pyrrolidines de formule (I), au moins un agent tensioactif.

Tensio-actifs

Le ou les agents tensioactifs peuvent être indifféremment choisis, seuls ou en mélanges, au sein des tensioactifs anioniques, amphotères, non ioniques, zwittérioniques et cationiques. Ils sont plus particulièrement choisis parmi les tensioactifs non ioniques.
Les tensioactifs convenant à la mise en oeuvre de la présente invention sont notamment les suivants :

(i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates; les alkyl ($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine. On notera que les alkylpolyglycosides constituent des tensio-actifs non-ioniques rentrant particulièrement bien dans le cadre de la présente invention tout comme les alcools gras éthoxylés.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.
Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :
$R_{34}$ -$CONHCH_2CH_2$ -$N(R_{35})(R_{36})(CH_2COO^-)$

dans laquelle : $R_{34}$ désigne un radical alkyle d'un acide $R_{34}$-COOH présent dans l'huile de coprah hydrolysée, un radical heptyle, nonyle ou undécyle, $R_{35}$ désigne un groupement bêta-hydroxyéthyle et $R_{36}$ un groupement carboxyméthyle ; et
$R_{34}'$-$CONHCH_2CH_2$-N(B)(C)
dans laquelle :
B représente -$CH_2CH_2OX'$, C représente -$(CH_2)_z$ -Y', avec z = 1 ou 2,
X' désigne le groupement -$CH_2CH_2$-COOH ou un atome d'hydrogène
Y' désigne -COOH ou le radical -$CH_2$- CHOH - $SO_3H$
$R_{34}'$ désigne un radical alkyle d'un acide $R_{37}$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, Sème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

(iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.
Les quantités d'agents tensioactifs présents dans la composition selon l'invention peuvent varier de 0,01 à 40% et de préférence de 0,1 à 30% du poids total de la composition.
Parmi les agents réducteurs utilisés pour conserver les colorants d'oxydation et utilisables dans la proportion de 0,05 à 1,5% en poids du poids total de la composition, on peut citer notamment le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone, et l'acide homogentisique, mais on préfère plus particulièrement utiliser la N-acétyl-L-cystéine décrite dans la demande de brevet de la demanderesse FR-9903829 ou un cétose et de préférence un cétohexose tel que le fructose comme décrit dans la demande de brevet de la demanderesse FR-9904338.
Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.
La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les 1-(4-aminophényl)-pyrrolidines de formule (I) selon l'invention et la ou les oxydo-réductases à 2 électrons ou à 4 électrons sont présents au sein de la même composition prête à l'emploi, et par conséquent ladite composition doit être exempte d'oxygène gazeux, de manière à éviter toute oxydation prématurée de la ou des 1-(4-aminophényl)-pyrrolidines de formule (I).

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres, à une température d'application comprise entre la température ambiante et 80°C, au moins une composition tinctoriale prête à l'emploi telle que définie précédemment, pendant un temps suffisant pour développer la coloration désirée. De façon préférentielle, les fibres sont ensuite rincées, on éventuellement lavées au shampooing, puis séchées.

La température d'application est de préférence comprise entre la température ambiante et 60°C et encore plus préférentiellement entre 35°C et 50°C.

Le temps suffisant au développement de la coloration sur les fibres kératiniques est généralement compris entre 1 et 60 minutes et encore plus précisément entre 5 et 30 minutes.

Selon une forme de réalisation particulière de l'invention, le procédé comporte une étape préliminaire consistant à stocker sous forme séparée, d'une part, une composition comprenant, dans un milieu approprié pour la teinture, au moins une 1-(4-aminophényl)-pyrrolidine de formule (I) telle que définie précédemment, et d'autre part, une composition renfermant, dans un milieu approprié pour la teinture, au moins une enzyme de type oxydo-réductase à 2 électrons ou à 4 électrons ou peroxydase, puis à procéder à leur mélange au moment de l'emploi avant d'appliquer ce mélange sur les fibres kératiniques.

Un autre objet de l'invention est un dispositif de teinture à plusieurs compartiments ou "kit" de teinture ou tout autre système de conditionnement à plusieurs compartiments dont au moins un premier compartiment renferme la composition (A) telle que définie ci-dessus et au moins un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## EXEMPLE 1

On a comparé une composition de teinture A prête à l'emploi selon l'invention, contenant comme précurseur de colorant d'oxydation le dichlorhydrate de 1-(4-aminophényl)-pyrrolidine, comme coupleur le dichlorhydrate de 1-β-hydroxyéthyloxy-2,4-diaminobenzène et comme oxydant de l'uricase (oxydase à 2 électrons) à une composition B de l'art antérieur contenant comme précurseur de colorant d'oxydation le sulfate de N,N-bis(β-hydroxyéthyl)-paraphénylènediamine.

Les compositions sont données ci-après.

Chacune des compositions a été appliquée sur des mèches de cheveux gris naturels à 90% de blancs, pendant 30 minutes à 40°C.

A l'issue du temps de pause, les mèches de cheveux ont été rincées, lavées avec un shampooing, puis séchées.

La couleur a ensuite été mesurée au colorimètre MINOLTA CM2002 dans le système L* a* b* .

Dans le système L* a* b* les 3 paramètres désignent respectivement l'intensité (L*), la nuance (a* ), et la saturation (b*).

Selon ce système, plus la valeur de L est élevée, plus la couleur est claire ou peu intense. Inversement, plus la valeur de L est faible, plus la couleur est foncée ou très intense.

a* et b* indiquent deux axes de couleurs, a* indique l'axe de couleur vert/rouge et b* l'axe de couleur bleu/jaune. Des valeurs proches de zéro pour a* et b* correspondent à des nuances grises.

La sélectivité de la coloration ΔE peut être calculée en appliquant l'équation suivante :

$$\Delta E = \sqrt{(L^* - L_o^*)^2 + (a^* - a_o^*)^2 + (b^* - b_o^*)^2}$$

Dans cette équation, ΔE représente la différence de couleur entre deux mèches, (dans le cas présent la sélectivité de la coloration) , L*, a*, et b* représentent respectivement l'intensité, la nuance et la saturation de la mèche de cheveux naturels teinte, $L_o^*$ , $a_o^*$ et $b_o^*$ représentant respectivement l'intensité, la nuance et la saturation de la mèche de cheveux permanentés teinte.

Plus la valeur de ΔE est importante, plus la différence de couleur entre les deux mèches est importante, et dans le cas présent, plus la sélectivité de la teinture est importante.

Les résultats ont été réunis dans le tableau (I) ci-dessous.

Composition A (invention)

| | |
|---|---|
| Dichlorhydrate de 1-(4-aminophényl)-pyrrolidine | 0,705 g |
| Dichlorhydrate de 1-β-hydroxyéthyloxy-2,4-diaminobenzène | 0,723 g |
| N-acétyl-L-cystéine | 0,10 g |
| Uricase | $20.10^3$ unitésU |
| Acide urique | 1 g |
| Monooléate de polyglycérol à 10 moles de glycérol | 1 g |
| Aculyn 22 (Rohm and Haas) | 0,75 g MA* |
| 2-amino-2-méthyl-1-propanol | q.s.p. pH 9,5 |
| Eau déminéralisée q.s.p | 100 g |

* désigne Matière Active

Composition B (art antérieur)

| | |
|---|---|
| Sulfate de N,N-bis(β-hydroxyéthyl)-paraphénylènediamine | 0,588 g |
| Dichlorhydrate de 1-β-hydroxyéthyloxy-2,4-diaminobenzène | 0,723 g |
| N-acétyl-L-cystéine | 0,10 g |
| Uricase | $20.10^3$ unitésU |
| Acide urique | 1 g |
| Monooléate de polyglycérol à 10 moles de glycérol | 1 g |
| Aculyn 22 (Rohm and Haas) | 0,75 g MA* |
| 2-amino-2-méthyl-1-propanol | q.s.p. pH 9,5 |
| Eau déminéralisée q.s.p | 100 g |

* désigne Matière Active

Tableau (I)

| Composition | L* | a* | b* | Sélectivité △E de la teinture |
|---|---|---|---|---|
| **A** (invention) | | | | |
| Cheveux naturels | 23.07 | -1.35 | -11.47 | **3.02** |
| Cheveux permanentés | 21.27 | -0.48 | -9.21 | |
| **B** (art antérieur) | | | | |
| Cheveux naturels | 35.62 | -5.53 | -7.37 | **11.61** |
| Cheveux permanentés | 25.10 | -2.27 | -11.04 | |

Ces résultats démontrent que la teinture selon l'invention (A) est plus puissante (L* plus bas) et moins sélective (ΔE plus petit) que celle de l'art antérieur (B).

EXEMPLE 2

On a comparé une composition de teinture A prête à l'emploi selon l'invention, contenant comme précurseur de colorant d'oxydation le dichlorhydrate de 1-(4-aminophényl)-pyrrolidine, comme coupleur le dichlorhydrate de 1-β-hydroxyéthyloxy-2,4-diaminobenzène et comme oxydant une laccase (oxydase à 4 électrons) à une composition B de

l'art antérieur contenant comme précurseur de colorant d'oxydation le sulfate de N,N-bis(β-hydroxyéthyl)-paraphénylènediamine.
Les compositions sont données ci-après.
Chacune des compositions a été appliquée sur des mèches de cheveux gris naturels à 90% de blancs, pendant 30 minutes à 40°C.
A l'issue du temps de pause, les mèches de cheveux ont été rincées, lavées avec un shampooing, puis séchées.
Les résultats ont été réunis dans le tableau (II) ci-dessous.

Composition A (invention)

| | |
|---|---|
| Dichlorhydrate de 1-(4-aminophényl)-pyrrolidine | 0,705 g |
| Dichlorhydrate de 1-β-hydroxyéthyloxy-2,4-diaminobenzène | 0,723 g |
| Carboxyméthylchitosane (OLEVASAN de la société SINOLION ) | 2 g MA* |
| Laccase | $30.10^6$ unitésU |
| Acide citrique | q.s.p. pH 7 |
| Eau déminéralisée q.s.p | 100 g |

Composition B (art antérieur)

| | |
|---|---|
| Sulfate de N,N-bis(β-hydroxyéthyl)-paraphénylènediamine | 0,588 g |
| Dichlorhydrate de 1-β-hydroxyéthyloxy-2,4-diaminobenzène | 0,723 g |
| Carboxyméthylchitosane (OLEVASAN de la société SINOLION ) | 2 g MA* |
| Laccase | $30.10^6$ unitésU |
| Acide citrique | q.s.p. pH 7 |
| Eau déminéralisée q.s.p | 100 g |

* désigne Matière Active

Tableau (II)

| Composition | L* |
|---|---|
| **A** (invention) | |
| Cheveux naturels ⇒ | 30.62 |
| Cheveux permanentés ⇒ | 22.28 |
| **B** (art antérieur) | |
| Cheveux naturels ⇒ | 37.23 |
| Cheveux permanentés ⇒ | 32.31 |

Ces résultats démontrent que la teinture selon l'invention (A) est plus puissante (L* plus bas) que celle de l'art antérieur (B).

## Revendications

1. Composition prête à l'emploi, pour la teinture d'oxydation des fibres kératiniques et en particulier des fibres kératiniques humaines telles que les cheveux, comprenant dans un milieu approprié pour la teinture, un système oxydant comprenant au moins une enzyme de type oxydo-réductase à 2 électrons ou à 4 électrons ou peroxydase, au moins un précurseur de colorant d'oxydation, **caractérisée par le fait que** le précurseur de colorant d'oxydation

est choisi parmi les 1-(4-aminophényl)-pyrrolidines de formule (I) suivante :

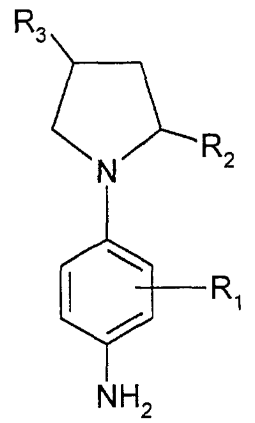

(I)

dans laquelle,

$R_1$ désigne un atome d'hydrogène, un radical alkyle en $C_1$-$C_6$, ou monohydroxyalkyle en $C_1$-$C_5$, ou polyhydroxyalkyle en $C_2$-$C_5$ ;
$R_2$ désigne un atome d'hydrogène, un radical -$CONH_2$, ou monohydroxyalkyle en $C_1$-$C_5$, ou polyhydroxyalkyle en $C_2$-$C_5$ ;
$R_3$ désigne un atome d'hydrogène, ou un radical OH ; et leurs sels d'addition avec un acide.

**2.** Composition selon la revendication 1, **caractérisée par le fait que** dans la formule (I), $R_1$, $R_2$, et $R_3$ désignent un atome d'hydrogène.

**3.** Composition selon la revendication 1, **caractérisée par le fait que** dans la formule (I), $R_1$ et $R_3$ désignent un atome d'hydrogène et $R_2$ désigne le radical-$CH_2OH$.

**4.** Composition selon la revendication 1, **caractérisée par le fait que** dans la formule (I), $R_1$ désigne un atome d'hydrogène, $R_2$ désigne le radical -$CH_2OH$ et R3 désigne le radical OH.

**5.** Composition selon la revendication 1, **caractérisée par le fait que** dans la formule (I), $R_1$ et $R_3$ désignent un atome d'hydrogène et $R_2$ désigne le radical -$CONH_2$.

**6.** Composition selon l'une quelconque des revendications précédentes, **caractérisée par le fait que** les sels d'addition avec un acide des 1-(4-aminophényl)-pyrrolidines de formule (I) sont choisis parmi les chlorhydrates, les bromhydrates, les sulfates, les tartrates, les lactates et les acétates.

**7.** Composition selon l'une quelconque des revendications précédentes, **caractérisée par le fait que** les 1-(4-aminophényl)-pyrrolidines de formule (I) et leurs sels d'addition avec un acide représentent de 0,001 à 10 % % en poids du poids total de la composition.

**8.** Composition selon la revendication 7, **caractérisée par le fait que** les 1-(4-aminophényl)-pyrrolidines de formule (I) et leurs sels d'addition avec un acide représentent de 0,01 à 8% en poids du poids total de la composition.

**9.** Composition selon la revendication 1, **caractérisée par le fait que** les oxydo-réductases à 2 électrons sont utilisées avec un donneur pour la ou lesdites enzymes et sont choisies parmi les pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.

**10.** Composition selon la revendication 1, **caractérisée par le fait que** les oxydo-réductases à 4 électrons sont choisies parmi les laccases, les tyrosinases, les catéchol oxydases et les polyphénols oxydases.

**11.** Composition selon la revendication 10, **caractérisée par le fait que** les oxydo-réductases à 4 électrons sont

choisies parmi les laccases d'origine végétale, d'origine animale, d'origine fongique ou d'origine bactérienne et parmi les laccases obtenues par biotechnologie.

**12.** Composition selon la revendication 11, **caractérisée par le fait que caractérisée par le fait que** la laccase est d'origine végétale et choisie parmi les laccases présentes dans les extraits d'Anacardiacées ; de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

**13.** Composition selon la revendication 10 ou 11, **caractérisée par le fait que** la laccase est d'origine fongique ou obtenue par biotechnologie.

**14.** Composition selon la revendication 13, **caractérisée par le fait que** la laccase est choisie parmi les laccases issues de Polyporus versicolor, de Rhizoctonia praticola, de Rhus vernicifera, de Scytalidium, de Polyporus pinsitus, de Myceliophtora thermophila, de Rhizoctonia solani, de Pyricularia orizae, de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

**15.** Composition selon la revendication 1, **caractérisée par le fait que** les peroxydases sont des peroxydases simplex.

**16.** Composition selon la revendication 1, **caractérisée par le fait que** la ou les peroxydases sont des catalases.

**17.** Composition selon la revendication 1, **caractérisée par le fait que** la ou les peroxydases sont utilisées avec un donneur pour la ou lesdites enzymes et sont choisies parmi les NADH peroxydases, les acides gras peroxydases, les NADPH peroxydases, les cytochrome-c peroxydases, les iodures peroxydases, les chlorures peroxydases, les L. ascorbates, les glutathion peroxydases .

**18.** Composition selon l'une quelconque des revendications 1 et 15 à 17, **caractérisée par le fait que** la ou les peroxydases sont d'origine animale, végétale, fongique, bactérienne ou obtenues par biotechnologie.

**19.** Composition selon la revendication 18, **caractérisée par le fait que** la ou les peroxydases sont issues de la pomme, de l'abricot, de l'orge, du radis noir, de la betterave, du chou, de la carotte, du maïs, du coton, de l'ail, du raisin, de la menthe, de la rhubarbe, du soja, de l'épinard, du coprin, du lait de bovin, des microorganismes du type Acetobacter peroxidans, Staphylococcus faecalis, Arthromyces ramosus.

**20.** Composition selon l'une quelconque des revendications précédentes, **caractérisée par le fait que** les oxydo-réductases à 2 électrons ou à 4 électrons représentent de 0,01 à 20 % en poids du poids total de la composition.

**21.** Composition selon la revendication 20, **caractérisée par le fait que** les oxydo-réductases à 2 électrons ou à 4 électrons représentent de 0,1 à 5 % en poids du poids total de la composition.

**22.** Composition selon l'une quelconque des revendications précédentes, **caractérisée par le fait que** la ou les peroxydases représentent de 0,0001 à 20% en poids du poids total de la composition.

**23.** Composition selon la revendication 22, **caractérisée par le fait que** la ou les peroxydases représentent de 0,001 à 10% en poids du poids total de la composition.

**24.** Composition selon l'une quelconque des revendications précédentes, **caractérisée par le fait qu'**elle renferme au moins un coupleur choisi parmi les méta-aminophénols, les métaphénylènediamines, les métadiphénols, les naphtols ou les coupleurs hétérocycliques, et leurs sels d'addition avec un acide.

**25.** Composition selon la revendication 24, **caractérisée par le fait que** les coupleurs sont choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène,

le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

**26.** Composition selon l'une quelconque des revendications 24 ou 25, **caractérisée par le fait que** le ou les coupleurs représentent de 0,0001 à 15% en poids du poids total de la composition.

**27.** Composition selon l'une quelconque des revendications précédentes, **caractérisée par le fait qu'**elle renferme au moins un colorant direct choisi parmi les nitrés, azoïques ou anthraquinoniques, neutres, cationiques ou anioniques dans la proportion pondérale de 0,001 à 20% et de préférence de 0,01 à 10% du poids total de la composition.

**28.** Composition selon l'une quelconque des revendications précédentes, **caractérisée par le fait que** le milieu approprié pour la teinture est constitué par de l'eau ou un mélange d'eau et d'au moins un solvant organique.

**29.** Composition selon la revendication 28, **caractérisée par le fait que** les solvant(s) sont présents dans des proportions comprises entre 1 et 40% en poids par rapport au poids total de la composition.

**30.** Composition selon l'une quelconque des revendications précédentes, **caractérisée par le fait qu'**elle renferme au moins un agent tensioactif non ionique dans la proportion d'au moins 0,01% en poids par rapport au poids total de la composition.

**31.** Composition selon l'une quelconque des revendications précédentes, **caractérisée par le fait qu'**elle renferme au moins un agent réducteur dans la proportion d'au moins 0,05% en poids par rapport au poids total de la composition.

**32.** Composition selon l'une quelconque des revendications précédentes, **caractérisée par le fait qu'**elle renferme au moins un polymère cationique ou amphotère dans la proportion d'au moins 0,01% en poids par rapport au poids total de la composition.

**33.** Composition selon l'une quelconque des revendications précédentes, **caractérisée par le fait qu'**elle présente un pH compris entre 3 et 11, et de préférence entre 4 et 9.

**34.** Composition selon l'une quelconque des revendications précédentes, **caractérisée par le fait qu'**elle se présente sous forme de liquide, de crème, de gel, éventuellement pressurisé, ou sous toute autre forme appropriée pour réaliser une teinture des fibres kératiniques.

**35.** Procédé de teinture d'oxydation des fibres kératiniques et en particulier des fibres kératiniques humaines telles que les cheveux, **caractérisé par le fait qu'**on applique sur lesdites fibres, à une température d'application comprise entre la température ambiante et 80°C, au moins une composition tinctoriale prête à l'emploi telle que définie dans l'une quelconque des revendications 1 à 34, pendant un temps suffisant pour développer la coloration désirée.

**36.** Procédé selon la revendication 35, **caractérisé par le fait que** la température d'application est comprise entre la température ambiante et 50°C

**37.** Procédé selon la revendication 35 ou 36, **caractérisé par le fait que** le temps suffisant au développement de la coloration est compris entre 1 et 60 minutes.

**38.** Procédé selon la revendication 37, **caractérisé par le fait que** le temps suffisant au développement de la coloration est compris entre 5 et 30 minutes.

**39.** Procédé selon l'une quelconque des revendications 35 à 38, **caractérisé par le fait qu'**il comporte une étape préliminaire consistant à stocker sous forme séparée, d'une part, une composition comprenant, dans un milieu approprié pour la teinture, au moins une 1-(4-aminophényl)-pyrrolidine de formule (I) telle que définie à l'une quelconque des revendications 1 à 8, et d'autre part, une composition renfermant, dans un milieu approprié pour la teinture, au moins une enzyme de type oxydo-réductase à 2 ou à 4 électrons ou peroxydase, puis à procéder à

leur mélange au moment de l'emploi avant d'appliquer ce mélange sur les fibres kératiniques.

**40.** Dispositif de teinture à plusieurs compartiments, **caractérisé par le fait qu'**il comporte au moins un premier compartiment renfermant une composition comprenant, dans un milieu approprié pour la teinture, au moins une 1-(4-aminophényl)-pyrrolidine de formule (I) telle que définie à l'une quelconque des revendications 1 à 8, et au moins un second compartiment renfermant une composition renfermant, dans un milieu approprié pour la teinture, au moins une enzyme de type oxydo-réductase à 2 ou à 4 électrons ou peroxydase.

Office européen des brevets

# RAPPORT DE RECHERCHE EUROPEENNE

Numéro de la demande
EP 01 40 0882

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A<br><br><br>D | EP 0 891 765 A (BRISTOL-MYERS SQUIBB COMPANY) 20 janvier 1999 (1999-01-20)<br>* le document en entier *<br>& US 5 851 237 A<br>22 décembre 1998 (1998-12-22)<br>--- | 1-40 | A61K7/13 |
| A,D | EP 0 310 675 A (KYOWA HAKKO KOGYO KK ET AL.) 12 avril 1989 (1989-04-12)<br>* le document en entier *<br>--- | 1-40 | |
| A | FR 2 773 478 A (L'OREAL)<br>16 juillet 1999 (1999-07-16)<br>* le document en entier *<br>----- | 1-40 | |
| | | | DOMAINES TECHNIQUES RECHERCHES (Int.Cl.7) |
| | | | A61K |

Le présent rapport a été établi pour toutes les revendications

| Lieu de la recherche | Date d'achèvement de la recherche | Examinateur |
|---|---|---|
| BERLIN | 16 juillet 2001 | Alvarez Alvarez, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03 82 (P04C02)

**ANNEXE AU RAPPORT DE RECHERCHE EUROPEENNE RELATIF A LA DEMANDE DE BREVET EUROPEEN NO.** EP 01 40 0882

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-07-2001

| Document brevet cité au rapport de recherche | Date de publication | Membre(s) de la famille de brevet(s) | Date de publication |
|---|---|---|---|
| EP 891765 A | 20-01-1999 | US 5851237 A | 22-12-1998 |
| EP 310675 A | 12-04-1989 | JP 7045385 B | 17-05-1995 |
| | | JP 63246313 A | 13-10-1988 |
| | | DE 3886867 D | 17-02-1994 |
| | | DE 3886867 T | 28-04-1994 |
| | | WO 8807360 A | 06-10-1988 |
| | | US 4961925 A | 09-10-1990 |
| FR 2773478 A | 16-07-1999 | AU 732926 B | 03-05-2001 |
| | | AU 1767799 A | 02-08-1999 |
| | | BR 9814920 A | 14-11-2000 |
| | | CN 1286618 T | 07-03-2001 |
| | | EP 1047379 A | 02-11-2000 |
| | | WO 9936037 A | 22-07-1999 |
| | | PL 341703 A | 23-04-2001 |

EPO FORM P0460

Pour tout renseignement concernant cette annexe : voir Journal Officiel de l'Office européen des brevets, No.12/82